Europäisches Patentamt

European Patent Office

Office européen des brevets

(11) Publication number: 0 253 486
A1

# (12) EUROPEAN PATENT APPLICATION

(21) Application number: 87304937.3

(22) Date of filing: 03.06.87

(51) Int. Cl.⁴: **A61K 7/22** , A61K 7/16

(30) Priority: 30.06.86 JP 154772/86

(43) Date of publication of application:
**20.01.88 Bulletin 88/03**

(84) Designated Contracting States:
**AT BE CH DE ES FR GB IT LI NL SE**

(71) Applicant: LION CORPORATION
3-7, Honjo 1-chome
Sumida-ku Tokyo(JP)

(72) Inventor: Ishii, Shigeru
6-1-1-1113, Ojima.
Koto-ku Tokyo(JP)
Inventor: Yamamoto, Mizuya
2047-2,Totsuka-cho Totsuka-ku
Yokohama-shi Kanagawa(JP)
Inventor: Yamazaki, Yoji
440-7, Tokunobu
Hiratsuka-shi Kanagawa(JP)

(74) Representative: Fisher, Bernard et al
Raworth, Moss & Cook 36 Sydenham Road
Croydon Surrey CR0 2EF(GB)

(54) Oral composition.

(57) An oral composition containing, as essential components, (i) at least one member selected from the group consisting of hydroxamic acids having the formula (I) and the salts thereof:

$$R - CH_2 - CO - NHOH \quad .......... \quad (I)$$

wherein R represents an alkyl group or an alkoxyphenyl group and (ii) at least one member selected from the group consisting of proteins, peptides, amino acids and the salts thereof as an astringent inhibitor.

This oral composition can effectively inhibit particular astringency caused by hydrohexamic acids or the salts thereof and has good foaming properties and a pleasant feeling upon application.

EP 0 253 486 A1

## ORAL COMPOSITION

BACKGROUND OF THE INVENTION

I. Field of the Invention

The present invention relates to an oral composition containing a hydroxamic acid having the formula (I)

$$R-CH_2-CO-NHOH \ldots\ldots (I)$$

wherein R represents an alkyl group or alkoxyphenyl group, or the salt thereof, which can inhibit the colonization of Streptococcus mutans in oral cavities and can provide an anticaries effect. More specifically, it relates to an oral composition capable of effectively preventing astringency when hydroxamic acid or the salt thereof is formulated, providing a pleasant feeling when applied, and having improved foaming properties.

2. Description of the Related Art

It has been noted in the art that Streptococcus mutans, among bacteria present in the oral cavity, causes caries. That is, Steptococcus mutans produces glucosyl transferases, which in turn, synthesize adhesive polysaccharides such as dextran and mutan. These polysaccharides form plaques including certain microflora, together with bacteria such as Streptococcus mutans therein. Streptococcus mutans, however, produces acids from various saccharide and these acids are retained in the plaques, and thus the enamel of the tooth is decalcified and caries occurs. Accordingly, to prevent the formation of caries, the deposition of Streptococcus mutans in the oral cavity must be inhibited.

Accordingly, various attempts have been made to inhibit the colonization of Streptococus mutans in the oral cavity, to thus prevent the formation of caries.

The present inventors previously found that hydroxamic acids having the above-mentioned formula (I) and the salts thereof are effective for inhibiting the deposition of Streptococcus mutans, and oral compositions containing the same have been proposed in Japanese Unexamined Patent Publication (Kokai) No. 61-126010 (U.S. Patent No. 4661324). However, oral compositions containing the hydroxamic acid having the above-mentioned formula (I) or the salt thereof formulated therein provide a particular astringency and, therefore, an improvement of these compositions is desired.

SUMMARY OF THE INVENTION

Accordingly, the objects of the present invention are to eliminate the above-mentioned disadvantages of the prior art and to provide an oral composition capable of effectively inhibiting a particular astringency caused by hydroxamic acids or the salts thereof and having good foaming properties and a pleasant feeling upon application.

Other objects and advantages of the present invention will be apparent from the following description.

In accordance with the present invention, there is provided an oral composition comprising, as essential components,

(i) at least one member selected from the group consisting of hydroxamic acids having the above-mentioned formula (I) and the salts thereof, and (ii) at least one member selected from the group consisting of proteins, peptides, amino acids, and the salts thereof, as an astringent inhibitor.

## DESCRIPTION OF THE PREFERRED EMBODIMENTS

The present inventors have found that, when proteins, peptides, amino acids and the salts thereof are used in combination with the hydroxamic acids and the salts thereof, the astringency caused by the use of the hydroxamic acids and the salts thereof in the oral composition can be effectively inhibited and, further more, the foaming properties are improved and the feeling upon application of the oral compositions containing the hydroxamic acids and the salts thereof are improved.

The oral compositions according to the present invention can be formulated as, for example, tooth paste, wet dentifrice, liquid dentifrice, mouth wash, gargle tablets, gingiva massage cream, or chewing gum, which contain one or more of hydroxamic acids having the above-mentioned formula (I) and the salts thereof.

These hydroxamic acids and the salts thereof effectively inhibit the colonization of Streptococcus mutans in an oral cavity, and in addition, inhibit gingival disease (or gingivitis). Thus, the oral composition according to the present invention has both an anticaries effect and preventive and therapeutic effects for periodontal diseases.

The hydroxamic acids preferably used in the present invention are those wheein R in the formula (I) represents an alkyl group with 5 to I7 carbon atoms, especially 7 to I3 carbon atoms and an alkoxyphenyl group with a $C_1$ to $C_8$ alkoxy group, especially a $C_1$ to $C_4$ alkoxy group. When R is an alkoxyphenyl group, R may be at either the ortho-, meta-, or para-position, but preferably, is at the para-position.

Typical examples of the hydroxamic acids preferably used in the present invention are 2-(4-butoxyphenyl)acethydroxamic acid (i.e. Bufexamac), 2-(4-methoxyphenyl)acethydroxamic acid, capryhydroxamic acid, and laurohydroxamic acid. Typical salts of the hydroxamic acids usable in the present invention are sodium, potassium, calcium, magnesium, and aluminum salts.

There are no critical limitations to the amounts of the hydroxamic acids having the formula (I) or the salts thereof to be formulated into the present oral composition, but the amount is preferably 0.005% to 5% by weight, more preferably 0.02% to I% by weight, based on the total weight of the composition. When the amount of the hydroxamic acid or the salts thereof is less than 0.005% by weight, the anticaries effect tends to become insufficient. Conversely, when the amount of the hydroxamic acid or the salts thereof is more than I% by weight, foreign tastes tend to be caused.

The present oral composition further contains one or more of proteins, peptides, amino acids, and the salts thereof as an astringent inhibitor for inhibiting astringency feelings caused by the hydroxamic acids or the salts thereof.

Examples of the proteins usable in the present invention are action, avidin, amandin, α-amylase, β-amylase, insulin, edestin, thioalbumin, ovomucoid, ovomucin, orosomucoid, catalase, canavalin, carboxypeptidase A and B, chymotrypsinogen A and B, clupeine, globulin, γ-globulin, globin, serum albumin, keratin, collagen, conalbumin, concanavalin, salmine, serine, tobacco mosaic virus, cytochrome thyroglobulin, transferrin, trypsinogen, trypsin, trypsin•inhibitor, nucleohistone, nucleoprotamine, peroxidase, haptoglobin, vitellin, vitellenin, phosuitin, phycoerythlin, phycocyan, fibrinogen, fibrin, fibroin, ferritin, plakalbumin, prothrombin, protamine, prolamine, pepsinogen, pepsin, hemoglobin, hemocyamin, Bence-Jones protein, hordein, macroglobulin, myoglobin, myogen, myosin, muramidase (lysozyme), methemoglobin, α-lactalbumin, β-lactalbumin, ricin, livetin, ribonuclease, livovittellin, and leukosine.

The peptides usable in the present invention include, for example, dipeptides, tripeptides, tetrapeptides, oligopeptides, polypeptides, and these peptides may be linear or cyclic. Typical examples of the peptides are the α-melancyte stimulating hormone, oxytocin, gramicidin S, and phalloidine.

Examples of the amino acids usable in the present invention are alanine, arginine, aspartic acid, aspara gine, cysteine, cystine, glutamic acid, glycine, histidine, oxylysine, oxyproline, isoleucine, leucine, lysine, methionine, phenylalanine, proline, oxyproline, serine, threonine, tryptophane, tyrosine, and valine.

Of these compounds, collagen, gelatin, alanine, albumin, glycine, and valine are preferable, and collagen, gelatine, and alamine are most preferable.

There are no critical limitations to the amounts of the astringent inhibitor to be formulated into the present oral composition, but preferably this amount is 0.I% to I0% by weight, more preferably 0.I% to 5% by weight, based on the total weight of the composition. When the amount of the astringent inhibitor is less than 0.I% by weight, the inhibition effect of the astringency is reduced. On the other hand, when the amount of the astringent inhibitor is more than I0% by weight, unwelcome taste effects are felt. The oral compositions according to the present invention may optionally contain, in addition to the above-mentioned essential constituents, any conventional ingredient; depending upon, for example, the kind of oral composition and the use thereof.

For example, in the case of dentrifices, abrasives such as calcium phosphate (dibasic)•dihydrate, calcium phosphate (dibasic)•anhyd, calcium phosphate (monobasic), calcium phosphate (tribasic), calcium carbonate, calcium pyrophosphate, insoluble sodium metaphospahte, amorphous slica, crystalline silica, aluminosilicate, aluminum hydroxide, aluminum oxide, magnesium phosphate (tribasic), magnesium carbonate, magnesium sulfate, titanium oxide, and resins can be included.

When pasty compositions such as toothpastes are prepared, binders such as sodium carboxymethyl cellulose, methyl cellulose, sodium carboxymethylhydroxyethyl cellulose, hydroxyethyl cellulose, sodium alginate, carageenan, gum arabic, xanthan gum, tragacanth gum, gum karaya, polyvinyl alcohol, sodium polyacrylate, carboxy vinyl polymer, and polyvinyl pyrrolidone, and humectants such as polyethylene glycol, ethylene glycol, sorbitol, glycerol, propylene glycol, l,3-butylene glycol, xylite, maltite, and lactite can be included.

Furthermore, the present oral composition may optionally contain one or more surfactants such as anionic surfactants including, for example, water-soluble salts of $C_8$-$C_{18}$ alkyl sulfates such as sodium lauryl sulfate and sodium myristyl sulfate, water-soluble higher fatty acid monoglyceride sulfates with a $C_{10}$-$C_{18}$ fatty acid residue such as sodium coconut fatty acid monoglyceride sulfate and sodium higher fatty acid monoglyceride monosulfate; $\alpha$-olefin sulfonates, paraffin sulfonates, sodium N-methyl-N-palmitoyl tauride, and sodium N-lauroyl-$\beta$-alanine; nonionic surfactants including, for example, fatty acid alkanol amides such as laurigl diethanolamide and myristyl diethanolamide, sucrose fatty acid esters such as sucrose mono and dilauryl esters and palmityl esters, polyoxyethylene sorbitan fatty acid esters, polyoxyethylene fatty acid esters, polyoxyethylene hydrogenated castor oil derivatives, lactose fatty acid esters, lactitol fatty acid esters, maltitol fatty acid esters, and polyoxyethylene polyoxypropylene block copolymers; cationic surfactants; and amphoteric suractants.

The present oral composition may further contain, as an optional ingredient, essential oils such as peppermint and spearmint; flavors such as flavour raw materials including $l$-menthol, carvone, eugenol, and anethole; sweeteners such as saccharin sodium, stebiocide, neohesperidyl dihydorchalcon, glycyrrhizn, perillartine, and p-methoxy cinnamic aldehyde; and preservatives. In addition to the compound having the formula (I), an effective component such as dextranase, protease, lytic enzymes, mutanase, sorbin acid, alexidine, $\beta$-glycyrrhetinic acid, hinokitiol, chlorohexidines, alkylglycines, alkyldiaminoethyl glycine salts, allantoin, $\epsilon$-aminocaproic acid, tranexamic acid, sodium monofluorophosphate, sodium fluoride, stannous fluoride, azulene, vitamin E, water-soluble monobasic or dibasic phosphates, quaternary ammonium compounds such as cetyl pyridinium chloride, sodium chloride, and plant extracts can be included.

When the other type oral compositions are prepared, any conventional ingredients may be used in the present oral compositions.

The oral compositions according to the present invention may be prepared in any conventional manner, for example, by mixing the above-mentioned essential and optional ingredients with water.

As disclosed in detail, according to the present invention, the astringency of the oral composition caused by the use of the hydroxamic acids or the salts thereof is effectively inhibited and the foaming properties of the oral composition are improved by using one or more of the above-mentioned proteins, peptides, amino acids and the salts thereof, in combination with the hydroxamic acid having the formula (I) or the salt thereof, in the oral composition. Thus, the feeling upon application of the resultant oral compositions is remarkably improved. Furthermore, since the present oral composition contains the hydroxamic acid or the salt thereof, the colonization of Streptococcus mutans in the oral cavity can be effectively inhibited and, therefore, the present oral composition is very effective for preventing caries and is also effective for preventing and curing periodontal diseases.

## EXAMPLES

The present invention will now be further illustrated by, but is by no means limited to, the following Experiments and Examples, wherein "%" means "% by weight" unless otherwise specified.

## Experiment I

A toothpaste composition having the following formulation was prepared by using, as the hydroxamic acid, 2-(4-butoxyphenyl)acethydroxamic acid. Various amino acids and the polymerization products thereof (e.g., proteins) were then formulated into the above toothpaste composition and the astringency inhibiting effects were organoleptically evaluated by a panel of l0 experts. The results are shown in Table I.

4

Formulation of Toothpaste

| Ingresident | % by weight |
|---|---|
| Calcium hydrogen phosphate dihydrate | 45.0 |
| Gelling silica | 2.5 |
| Sorbitol | 20.0 |
| Propylene glycol | 2.5 |
| Sodium carboxymethyl cellulose | 1.2 |
| Sodium lauryl sulfate | 1.5 |
| Sodium saccharinate | 0.1 |
| 2-(4-Butoxyphenyl)acethydroxamic acid | 0.05 |
| Flavour | 1.0 |
| Amino acid or polymer thereof (Protein) | 0.1-0.5 |
| Purified water | Balance |

100

Table 1

| Sample No. | Amino acid or its polymer | Amount (%) | Effect [*1] |
|:---:|---|:---:|:---:|
| 1 | Collagen | 0.1 | ++ |
| 2 | Gelatine (MW[*]: more than 100000) | 0.3 | ++ |
| 3 | Gelatine (MW: 10000) | 0.5 | ++ |
| 4 | Alanine | 0.5 | ++ |
| 5 | Albumin (egg white) | 0.5 | + |
| 6 | Albumin (serum) | 0.5 | + |
| 7 | Glycine | 0.5 | + |
| 8 | Sodium glutamate | 0.5 | ± |
| 9 | Sodium asparaginate | 0.5 | ± |
| 10 | Valine | 0.5 | + |

[*1]: Evaluation Standard for Astringency Inhibition Effect

The astringency of the oral composition was evaluated by comparing the astringency o the oral composition with a composition having the same ingredients as mentioned above except that it contained neither amino acids nor the polymers thereof (proteins). The evaluation standards were as follows:

+ + : Very effective

+ : Effective

± : Slightly effective

- : Not effective (No difference)

As is clear from the results shown in Table I, the toothpastes containing amino acids and proteins have an excellent astringency inhibiting effect comparison with the toothpastes not containing hydroxamic acid.

Experiment II

The dentrifices A, B, and C having the formulations shown in Table 2 were prepared by using 2-(4-butoxyphenyl)acethydroxamic acid as the hydroxamic acid. The astringency inhibition effects of gelatin and alanine were organoleptically evaluated by a panel consisting of 60 members, using the Scheffe's paired comparison method.

The results are shown in Table 3.

6

## Table 2

| Ingredient | A | B | C |
| --- | --- | --- | --- |
| Calcium phosphate (dibasic)·dihydrate | 44.0% | 44.0% | 44.0% |
| Gelling silica | 3.0 | 3.0 | 3.0 |
| Sorbitol | 20.0 | 20.0 | 20.0 |
| Propylene glycol | 2.5 | 2.5 | 2.5 |
| Sodium carboxymethyl cellulose | 1.0 | 1.0 | 1.0 |
| Carageenan | 0.3 | 0.3 | 0.3 |
| Sodium lauryl sulfate | 1.5 | 1.5 | 1.5 |
| Sodium saccharinate | 0.1 | 0.1 | 0.1 |
| 2-(4-Butoxyphenyl)acethydroxamic acid | 0.05 | 0.05 | 0.05 |
| Gelatin | – | 0.3 | – |
| Alamine | – | – | 0.5 |
| Flavor | 1.0 | 1.0 | 1.0 |
| Purified water | Balance | Balance | Balance |
| Total | 100.0% | 100.0% | 100.0% |

Table 3

| Items | Average Taste Degree[1] | | |
|---|---|---|---|
| | A | B | C |
| Dispersibility | −0.17 | 0.13 | 0.03 |
| Foaming property[2] | −0.28 | 0.15 | 0.13 |
| Astringency[2] | −0.32 | 0.17 | 0.15 |
| Refreshing effect | −0.10 | 0.05 | 0.05 |

*1: Score
+2: Very good
+1: Good
 0: Same
−1: Poor
−2: Very poor
*2: Significant to a 1% level

As is clear from the results shown in Table 3, gelatin and alanine remarkably improve the foaming properties and the astrigency of the toothpaste containing hydroxamic acid and provide a good dispersibility and refreshing effect of the toothpastes.

The following Examples illustrate the formulations of the oral compositions according to the present invention, but in no way limit the present invention.

Example 1: Toothpaste

| Ingredient | % |
|---|---|
| Calcium phosphate (dibasic)·dihydrate | 45 |
| Silicic anhydride | 3 |
| Sodium carboxymethyl cellulose | 1.1 |
| Carageenan | 0.2 |
| Sorbitol | 20.0 |
| Propylene glycol | 3.0 |
| Sodium $C_{10}$-$C_{16}$ alkyl* sulfate | 1.5 |
| Chlorohexidine hydrochloride | 0.01 |
| 2-(4-Butoxyphenyl)acethydroxamic acid | 0.05 |
| Collagen | 0.1 |
| Paraben | 0.2 |
| Sodium saccharinate | 0.1 |
| Flavor | 1.1 |
| Purified water | Balance |
| | 100.0% |

*$C_{10}$-$C_{16}$ alkyl sulfate
* $C_{10}$: 0-20%
$C_{12}$: 50-80%
$C_{14}$: 10-30%
$C_{16}$: 0-15%

98% or more of the sulfate has the above carbon distribution (the same hereinbelow).

Example 2:  Toothpaste

| Ingredient | % |
| --- | --- |
| Calcium phosphate (dibasic)·dihydrate | 25 |
| Calcium phosphate (dibasic)·anhydrate | 20 |
| Silicic anhydride | 2.5 |
| Sodium carboxymethyl cellulose | 1.0 |
| Carageenan | 0.3 |
| Sorbitol | 5 |
| Glycerol | 15 |
| Propylene glycol | 2.5 |
| Sodium lauryl sulfate | 1.8 |
| Lauric diethanolamide | 0.1 |
| Tocopherol acetate | 0.1 |
| 2-(4-Butoxyphenyl)acethydroxamic acid | 0.1 |
| Gelatin | 0.5 |
| Alanine | 0.1 |
| Sodium benzoate | 0.2 |
| Sodium saccharinate | 0.15 |
| Flavor | 1.0 |
| Purified water | Balance |
|  | 100.0% |

Example 3:  Toothpaste

| Ingredient | % |
| --- | --- |
| Aluminum hydroxide | 45 |
| Carageenan | 0.4 |
| Sodium alginate | 0.3 |
| Sodium polyacrylate | 0.1 |
| Glycerol | 20 |
| Polyethylene glycol #400 | 3.0 |
| Sodium $C_{10}$-$C_{16}$ alkylsulfate | 1.5 |
| Myristyl diethanolamide | 1.0 |
| β-Glycyrrhetinic acid | 0.1 |
| Dextranase (1,000,000 unit/g) | 0.2 |
| Sodium monofluorophosphate | 0.76 |
| Laurohydroxamic acid | 0.1 |
| Alanine | 0.5 |
| Paraben | 0.1 |
| Sodium benzoate | 0.1 |
| Stehiocide | 0.1 |
| Flavor | 1.1 |
| Purified water | Balance |

100.0%

Example 4:  Toothpaste

| Ingredient | % |
|---|---|
| Calcium phosphate (dibasic)·dihydrate | 12 |
| Calcium phosphate (dibasic)·anhydrate | 30 |
| Silicic anhydride | 2.5 |
| Sodium carboxymethyl cellulose | 1.5 |
| Sorbitol | 21 |
| Polyethylene glycol #400 | 3.0 |
| Sodium lauryl sulfate | 1.5 |
| Sucrose lauric acid monoester | 0.3 |
| Tranexamic acid | 0.05 |
| 2-(4-Butoxyphenyl) acethydroxamic acid | 0.05 |
| Gelatin | 0.3 |
| Valine | 0.1 |
| Paraben | 0.1 |
| Sodium saccharinate | 0.1 |
| Flavor | 1.0 |
| Purified water | Balance |

100.0%

Example 5:  Toothpaste

| Ingredient | % |
| --- | --- |
| Silicic anhydride | 30 |
| Sodium carboxymethyl cellulose | 1.1 |
| Sodium polyacrylate | 0.1 |
| Glycerol | 25 |
| Polyethylene glycol #400 | 4.0 |
| Sodium $C_{10}$-$C_{16}$ alkylsulfate | 1.2 |
| Sodium lauroyl sarcosinate | 0.3 |
| Chlorohexidine gluconate | 0.01 |
| Sodium fluoride | 0.21 |
| Dipotassium glycyrrhetinic acid | 0.1 |
| Carylhydroxamic acid | 0.1 |
| Phenylalanine | 0.5 |
| Paraben | 0.05 |
| Sodium saccharinate | 0.1 |
| Flavor | 0.1 |
| Purified water | Balance |

100.0%

Example 6:   Toothpaste

| Ingredient | % |
|---|---|
| Calcium phosphate (dibasic)·dihydrate | 25 |
| Calcium phosphate (dibasic)·anhydrate | 25 |
| Silicic anhydride | 2.5 |
| Sodium carboxymethyl cellulose | 1.1 |
| Carageenan | 0.2 |
| Glycerol | 20 |
| Propylene glycol | 3.0 |
| Sodium $C_{10}$-$C_{16}$ alkylsulfate | 1.5 |
| Sucrose palmitic acid monoester | 0.3 |
| ε-Aminocaproic acid | 0.1 |
| Allantoinate | 0.1 |
| 2-(4-Butoxyphenyl)acethydroxamic acid | 0.1 |
| Gelatin | 0.3 |
| Valine | 0.1 |
| Paraben | 0.2 |
| Sodium saccharinate | 0.18 |
| Flavor | 1.0 |
| Purified water | Balance |

## Example 7: Toothpaste

| Ingredient | % |
| --- | --- |
| Silicic anhydride | 26 |
| Sodium carboxymethyl cellulose | 1.2 |
| Sodium polyacrylate | 0.2 |
| Sorbitol | 40 |
| Sodium lauryl sulfate | 1.5 |
| Lauryl diethanolamide | 0.3 |
| Chlorohexidine hydrochloride | 0.01 |
| Sodium monofluorophospahte | 0.76 |
| 2-(4-Butoxyphenyl)acethydroxamic acid | 0.2 |
| Collagen | 0.3 |
| Paraben | 0.1 |
| Sodium benzoate | 0.1 |
| Sodium saccharinate | 0.05 |
| Flavor | 1.1 |
| Purified water | Balance |

100.0%

Example 8:  Toothpaste

| Ingredient | % |
| --- | --- |
| Aluminum hydroxide | 42 |
| Silicic anhydride | 3.0 |
| Sodium carboxymethyl cellulose | 1.2 |
| Carageenan | 0.3 |
| Sorbitol | 10 |
| Glycerol | 12 |
| Sodium $C_{10}$-$C_{16}$ alkyl sulfate | 1.8 |
| β-Glycyrrhetinic acid | 0.1 |
| 2-(4-Butoxyphenyl)acethydroxamic acid | 0.1 |
| Gelatin | 0.5 |
| Paraben | 0.2 |
| Sodium saccharinate | 0.1 |
| Flavor | 1.0 |
| Purified water | Balance |

100.0%

Example 9:  Liquid dentifrice

| Ingredient | % |
|---|---|
| Silicic anhydride | 2.0 |
| Sodium carboxymethyl cellulose | 1.0 |
| Glycerol | 3.0 |
| Polyethylene glycol #400 | 5 |
| Sodium $C_{10}$-$C_{16}$ alkylsulfate | 1.5 |
| Lauryl diethanolamide | 0.5 |
| 2-(4-Butoxyphenyl) acethydroxamic acid | 0.1 |
| Collagen | 0.2 |
| Albumin (egg white) | 0.1 |
| Paraben | 0.1 |
| Sodium saccharinate | 0.05 |
| Flavor | 1.1 |
| Purified water | Balance |

100.0%

Example 10:  Wet dentifrice

| Ingredient | % |
| --- | --- |
| Aluminum hydroxide | 80 |
| Glycerol | 10 |
| Sodium $C_{10}$-$C_{16}$ alkylsulfate | 1.0 |
| Dipotassium glycyrrhetinate | 0.1 |
| 2-(4-Butoxyphenyl)acethydroxamic acid | 0.05 |
| Alanine | 0.5 |
| Aspartame | 0.1 |
| Flavor | 1.0 |
| Purified water | Balance |

100.0%

## Example 11: Oral pasta

| Ingredient | % |
|---|---|
| Cetyl alcohol | 15.0 |
| Squalane | 15.0 |
| Silicic anhydride | 5.0 |
| Polyoxyethylene (20 mol) hydrogenated castor oil | 0.2 |
| Sorbitan monooleyl ester | 1.0 |
| Dipotassium glycyrrhetinate | 0.1 |
| Sodium saccharinate | 0.2 |
| Flavor | 0.3 |
| 2-(4-Butoxyphenyl)acethydroxamic acid | 0.2 |
| Alanine | 0.5 |
| Purified water | Balance |

100.0%

Example 12: Mouthwash

| Ingredient | % |
| --- | --- |
| Glycerol | 10.0 |
| Ethyl alcohol | 15.0 |
| Polyoxyethylene (40 mol) hydrogenated castor oil | 1.0 |
| Chlorohexidine hydrochloride | 0.01 |
| Sodium saccharinate | 0.1 |
| Flavor | 0.2 |
| 2-(4-Butoxyphenyl)acethydroxamic acid | 0.1 |
| Glycine | 0.5 |
| Purified water | Balance |
|  | 100.0% |

**Claims**

1. An oral composition comprising (i) at least one member selected from the group consisting of hydroxamic acids of formula (I) and salts thereof:

R-CH$_2$-CO-NHOH ........... (I)

wherein R represents an alkyl group or an alkoxyphenyl group, and (ii) at least one member selected from the group consisting of proteins, peptides, amino acids, and salts thereof as an astringent inhibitor.

2. An oral composition as claimed in claim I, wherein said hydroxamic acid of the formula (I) is 2-(4-butoxyphenyl)acethydroxamic acid, 2-(4-methoxyphenyl)acethydroxamic acid, caprylhydroxamic acid, or laurohydroxamic acid.

3. An oral composition as claimed in claim I or 2, wherein the formulating amount of the hydroxamic acid of the formula (I) or the salt thereof is 0.005% to 5% by weight, based on the total weight of the composition.

4. An oral composition as claimed in claim I, 2, or 3, wherein the protein is collagen or gelatin and the amino acid is alanine.

5. An oral composition as claimed in anyone of claims I to 4, wherein the formulating amount of the component (ii) is 0.I% to I0% by weight, based on the total weight of the composition.

6. An oral composition as claimed in claim I, wherein R in the formula (I) is an alkyl group having 5 to I7 carbon atoms.

7. An oral composition as claimed in claim I, wherein R in the formula (I) is an alkoxyphenyl group having a C$_1$ to C$_8$ alkoxy group.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X,D | DE-A-3 541 025 (LION CORP.)<br><br>* Claims 1-3; page 8, lines 13-22 *<br><br>--- | 1-3,5-7 | A 61 K 7/22<br>A 61 K 7/16 |
| A | US-A-3 427 381 (J.J. KIRKLAND)<br><br>* Whole document *<br><br>--- | 1-3,6-7 | |
| A | GB-A-2 122 491 (LION CORP.)<br>* Page 3, right-hand column, lines 88-127 *<br><br>----- | 1,4-5 | |

TECHNICAL FIELDS SEARCHED (Int. Cl.4)

A 61 K

The present search report has been drawn up for all claims

| Place of search | Date of completion of the search | Examiner |
|---|---|---|
| THE HAGUE | 04-11-1987 | FISCHER J.P. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82